# EUROPEAN PATENT APPLICATION

(11) **EP 2 196 129 A1**
(43) Date of publication of application: **16.06.2010**
(21) Application number: 08828988.9
(22) Date of filing: 05.09.2008
(51) Int. Cl.: A61B 1/00, A61B 5/07, G02B 23/24

(54) **IN-VIVO INFORMATION ACQUIRING DEVICE AND POWER SUPPLY CONTROL METHOD**

(30) Priority: 07.09.2007 JP 2007233322
(71) Applicant: Olympus Medical Systems Corp., Tokyo 151-0072 (JP)
(72) Inventor: KIMOTO, Seiichiro, Tokyo 151-0072 (JP)
(74) Representative: Gunzelmann, Rainer
(86) International application number: PCT/JP2008/066049
(87) International publication number: WO 2009/031640

(57) **Abstract**

A power-on signal detector (191) detects an output pattern of a control signal from a magnetic switch (180) while the power is off. When the output pattern is identical with an on pattern, the power-on signal detector (191) outputs a detection signal to a power supply control unit (170). When the output pattern is identical with an off pattern, the power-off signal detector (193) outputs a detection signal to the power supply control unit (170). the power supply control unit (170) switches between the state where a drive power from a power unit (160) is supplied and the state where the drive power from the power unit (160) is cut off.

## Description

### TECHNICAL FIELD

The present invention relates to an in-vivo information acquiring apparatus that is to be introduced into a subject and obtains in-vivo information about the interior of the body of a subject, and to a power supply control method performed by the in-vivo information acquiring apparatus.

### BACKGROUND ART

In the field of endoscopes, recently, swallowable capsule endoscopes, which include a capsule-shaped case containing an imaging unit that acquires information about in-vivo images of a subject; an illuminating unit that illuminates a site whose image is to be picked up by the imaging unit; and a transmitting unit that wirelessly transmits the image information acquired by the imaging unit, have been proposed. The capsule endoscope is to be swallowed by a patient who is a subject and introduced into the subject. Until being naturally excreted, the capsule endoscope sequentially captures images of the body cavities while moving in the body cavities depending on the peristalsis, and wirelessly transmits acquired image information to the outside of the body.

To control driving the capsule endoscope, a configuration is proposed in which a reed switch for on/off with a magnetic signal is provided in the capsule endoscope, and in which a package in which the capsule endoscope is housed includes a permanent magnet for providing a magnetic field. According to the technology, the capsule endoscope is not driven while being housed in the package. When the capsule endoscope is taken out of the package and not influenced by the permanent magnet, driving the capsule endoscope is started (see, International Publication No. 01/35813).

On the other hand, a capsule endoscope is recently proposed in which a magnet provided in the capsule endoscope is rotated by externally applying a revolving magnetic field, so that the capsule endoscope is rotated and guided in a body.

Patent Document 1: International Publication No. 01/35813 Pamphlet

### DISCLOSURE OF INVENTION

### PROBLEM TO BE SOLVED BY THE INVENTION

There is a demand that the capsule endoscope is configured to supply and cut off the power after it is introduced into the subject because a finite power source such as a battery is used in the capsule endoscope and thus its power consumption needs to be minimized. For example, the technology disclosed in Patent Document 1 allows the power to be supplied and cut off outside the subject by moving a magnet close to or apart from the capsule endoscope that has been introduced into the subject to operate a reed switch. In the capsule endoscope that has a function of guiding the capsule endoscope by the revolving magnetic field externally applied as described above, for example, however, there is a problem that an external magnetic field for induction makes a malfunction in the reed switch, thereby supplying and cutting off the power incorrectly. A method for switching supplying and cutting off the power of the capsule endoscope without detecting a magnetic signal may switch supplying and cutting off the power source according to an instruction for supplying or cutting off the power received by a receiving unit provided in the capsule endoscope. However, such additional receiving unit is required and this is not preferable.

The present invention is made in view of the aforementioned conventional problems, and an object of the present invention is to provide an in-vivo information acquiring apparatus and a power supply control method capable of assuredly switching supplying and cutting off the power in the in-vivo information acquiring apparatus having been introduced into the subject with the simple configuration.

### MEANS FOR SOLVING PROBLEM

To solve the problems and achieve the object, an in-vivo information acquiring apparatus according to the present invention includes an information acquiring unit that acquires in-vivo information; a power source that supplies a power to the information acquiring unit; a magnetic sensor unit that detects a magnetic signal externally input to the magnetic sensor unit, and outputs a control signal corresponding to a state in which the magnetic signal is detected; a pulse number counting unit that counts the number of pulses of pulse signals from the magnetic sensor unit; a pulse number determining unit that determines whether the number of pulses counted by the pulse number counting unit is not smaller than a predetermined number; and a power cut control unit that switches a state in which the power from the power source is supplied to the information acquiring unit to a state in which the power from the power source to the information acquiring unit is cut off.

The in-vivo information acquiring apparatus according to the present invention further includes an interval detecting unit that detects an interval between outputs of pulse signals from the magnetic sensor, wherein the pulse number counting unit updates the number of outputs of pulse signals when the interval detected by the interval detecting unit does not exceeds a predetermined reference interval.

In the in-vivo information acquiring apparatus according to the present invention, the pulse number determining unit determines whether the number of pulses counted by the pulse number counting unit is not smaller than the predetermined number at timing at which the interval detected by the interval detecting unit reaches the predetermined reference interval.

In the in-vivo information acquiring apparatus according to the present invention, the interval detecting unit includes a counter.

The in-vivo information acquiring apparatus according to the present invention further includes a pattern determining unit that determines a pattern of pulse signals input to the pattern determining unit; and a power supply control unit that switches the state in which the power from the power source to the information acquiring unit is cut off to the state in which the power from the power source is supplied to the information acquiring unit when a predetermined pattern of pulse signals is input to the pattern determining unit.

In the in-vivo information acquiring apparatus according to the present invention, the pattern is a single pulse.

In the in-vivo information acquiring apparatus according to the present invention, the magnetic sensor includes a magnetic switch.

A power supply control method according to the present invention is performed by an in-vivo information acquiring apparatus including an information acquiring unit that acquires in-vivo information and a power source that supplies a power to the information acquiring unit, and includes detecting a magnetic signal externally input; outputting a control signal corresponding to a state in which the magnetic signal is detected; counting the number of pulse signals; determining whether the number of pulse signals is not smaller than a predetermined number; and switching a state in which the power from the power source is supplied to a state in which the power from the power source is cut off when the number of pulse signals is not smaller than the predetermined number.

### EFFECT OF THE INVENTION

According to the present invention, when the number of pulse signals output from the magnetic sensor unit is not smaller than a predetermined value, switching is performed between the state in which the power from the power source is supplied and the state in which the power from the power source is cut off. In other words, as long as the number of pulse signals output from the magnetic sensor unit is smaller than the predetermined value, switching is not performed between the state in which the power from the power source is supplied and the state in which the power from the power source is cut off. This prevents erroneously switching between supplying and cutting off the power even under the environment where an external magnetic field such as a revolving magnetic field is appropriately applied to guide the in-vivo information acquiring apparatus. Accordingly, supplying and cutting off the power in the in-vivo information acquiring apparatus having been introduced into the subject can be assuredly switched with the simple configuration.

### BRIEF DESCRIPTION OF DRAWINGS

FIG. 1 is a schematic diagram of a structure of a capsule endoscope.
FIG. 2 is a block diagram of a functional configuration of the capsule endoscope.
FIG. 3 is a timing chart for explaining an operation of a control signal detecting unit according to a first embodiment.
FIG. 4 is a block diagram of a functional configuration of a capsule endoscope according to a second embodiment.
FIG. 5 is a timing chart for explaining an operation of a power-off signal detector according to the second embodiment.
FIG. 6 is another timing chart for explaining the operation of the power-off signal detector according to the second embodiment.
FIG. 7 is still another timing chart for explaining the operation of the power-off signal detector according to the second embodiment.
FIG. 8 is a flowchart for explaining a flow of an operation for cutting off the power performed by the capsule endoscope according to the second embodiment.
FIG. 9 is a schematic diagram of a structure of a capsule endoscope including two pairs of an imaging unit and an illuminating unit.

- 100: Capsule endoscope
- 110: Imaging unit
- 120: Illuminating unit
- 130: Signal processing unit
- 140: Transmission processing unit
- 143: Transmitting antenna
- 150: Operation control unit
- 160: Power unit
- 170: Power supply control unit
- 180: Magnetic switch
- 190: Control signal detecting unit
- 191: Power-on signal detector
- 193: Power-off signal detector

### BEST MODE(S) FOR CARRYING OUT THE INVENTION

Preferred embodiments of an in-vivo information acquiring apparatus according to the present invention are explained in detail below with reference to the accompanying drawings. As an example of the in-vivo information acquiring apparatus, the case is explained below where the in-vivo information acquiring apparatus is applied to a capsule endoscope that is introduced into a subject and acquires information about picked-up images of the body cavities.

### First Embodiment

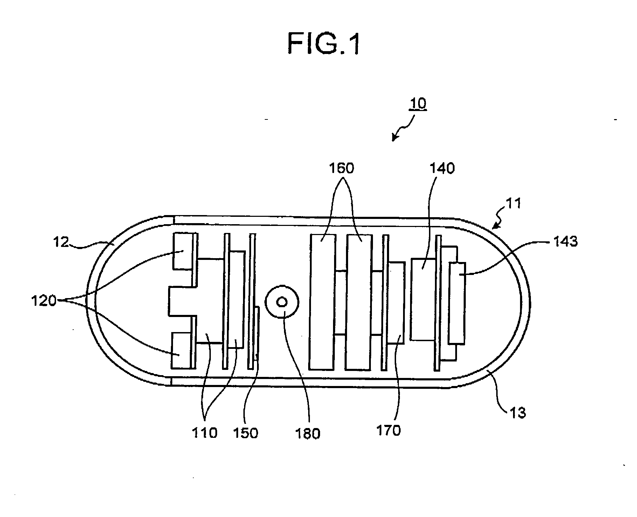

First, a configuration of a capsule endoscope according to a first embodiment is explained. FIG. 1 is a schematic diagram of a structure of a capsule endoscope 10. The capsule endoscope 10 exemplarily shown has an image pickup function and a wireless function. The capsule endoscope 10 is swallowed from the mouse of a subject such as a human or an animal and introduced into to the body for, for example, examination. Until being naturally excreted, the capsule endoscope 10 sequentially captures images of, for example, the esophagus, the stomach, the small intestine, and the colon to acquire image information about the images, and wirelessly transmits the acquired image information to the outside of the body. The wirelessly transmitted image information is received by a receiving device arranged outside the body, and the images are displayed on, for example, a display and observed.

Specifically, the capsule endoscope 10 includes a capsule-shaped casing 11 containing an imaging unit 110 that captures images of the body cavities and acquires image information thereof; an illuminating unit 120 that illuminates the body cavities with illuminating light; a transmission processing unit 140 that performs processing for wirelessly transmitting the image information, which is acquired by the imaging unit 110, via a transmitting antenna 143; an operation control unit 150 that performs operation control on each unit of the apparatus; a power unit 160 that is a power source that supplies a drive power to each unit of the capsule endoscope 10; a magnetic switch 180 that is a magnetic sensor unit for switching between the state in which a drive power from the power unit 160 is supplied and the state in which the drive power from the power unit 160 is cut off; and a power supply control unit 170 that controls supplying the drive power to each unit of the apparatus.

The casing 11 has a size that can be swallowed by a human. The casing 11 is formed by connecting an approximately substantially dome-shaped front cover 12 and a body cover 13. The front cover 12 is formed of a transparent member, and it functions as an optical window. Specifically, in the casing 11, the imaging unit 110 and the illuminating unit 120 are opposed to the front cover 12. The front cover 12 allows the illuminating light from the illuminating unit 120 to reach the outside of the casing 11 and guides the reflected light into the casing 11.

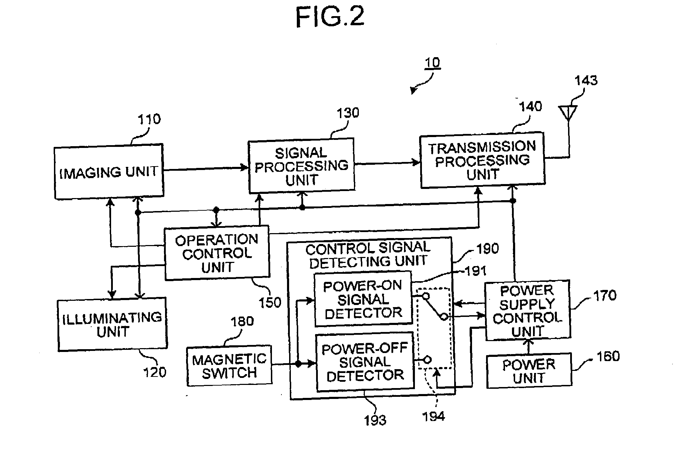

FIG. 2 is a block diagram of a functional configuration of the capsule endoscope 10. As shown in FIG. 2, the capsule endoscope 10 includes the imaging unit 110, the illuminating unit 120, a signal processing unit 130, the transmission processing unit 140, the transmitting antenna 143, the operation control unit 150, the power unit 160, the power supply control unit 170, the magnetic switch 180, and a control signal detecting unit 190.

The imaging unit 110 includes an imaging device including an image sensor, such as a CCD or a CMOS, and an imaging lens that forms an image of incident light on the imaging device. The imaging unit 110 performs a capturing operation for capturing images of the interior of the subject by outputting an analog signal corresponding to the intensity of the incident light. Specifically, the imaging unit 110 performs the capturing operation at timing at which an imaging unit drive pulse is supplied from the operation control unit 150.

The illuminating unit 120 includes a light emitting device, such as an LED, and a circuit for driving the light emitting device. The illuminating unit 120 performs an illuminating operation for illuminating with illuminating light a site whose image is to be captured by the imaging unit 110. Specifically, the illuminating unit 120 performs the illuminating operation at timing at which an illuminating unit drive pulse is supplied from the operation control unit 150.

The signal processing unit 130 performs required processing on the image information acquired by the imaging unit 110 and generates a transmission signal. Specifically, after performing analog signal processing, i.e., correlated double sampling, on an analog signal input from the imaging unit 110, the signal processing unit 130 converts the analog signal into a digital signal. Based on the obtained digital signal, the signal processing unit 130 generates a transmission signal for wirelessly transmit the image information to the outside. For example, the signal processing unit 130 generates a transmission signal in a way that information about one image is generated as one frame, a vertical synchronization signal is added to the top of the frame, and a horizontal synchronization signal is added to the top of component data of each line. The signal processing unit 130 outputs the transmission signal to the transmission processing unit 140. The transmission signal is input to the transmission processing unit 140 and wirelessly transmitted to an external receiving device. The receiving device having received the transmission signal processes each image signal by detecting the top of the image based on the vertical synchronization signal and detecting the top of the image signal of each line based on the horizontal synchronization signal, thereby obtaining the image information.

The transmission processing unit 140 includes a transmission circuit that performs modulation processing etc. on the transmission signal input from the signal processing unit 130 if necessary, thereby generating a wireless signal. The transmission processing unit 140 wirelessly transmits the wireless signal to the outside via the transmitting antenna 143.

The operation control unit 150 controls each unit of the capsule endoscope 10, and generally controls operations of the capsule endoscope 10. For example, the operation control unit 150 performs processing for controlling the image pickup operation of the imaging unit 110 and the lighting operation of the illuminating unit 120 by generating timing at which the imaging unit 110 and the illuminating unit 120 are driven. Specifically, the operation control unit 150 controls the capturing operation of the imaging unit 110 by supplying the imaging unit drive pulse at predetermined intervals, and controls the illuminating operation of the illuminating unit 120 by supplying the illuminating unit drive pulse just before supplying a capturing start pulse. The operation control unit 150 drives the signal processing unit 130 based on the timing at which the imaging unit drive pulse is supplied, and synchronizes the processing performed by the signal processing unit 130 with the timing at which the imaging unit drive pulse is supplied.

The power supply control unit 170 serves as a power cut control unit and a power supply control unit. The power supply control unit 170 switches between the state in which the drive power from the power unit 160 is supplied and the state in which the drive power from the power unit 160 is cut off (i.e., power on/off). While the power is on, the power supply control unit 170 distributes the drive power supplied from the power unit 160 to the units of the capsule endoscope 10, such as the imaging unit 110, the illuminating unit 120, the signal processing unit 130, the transmission processing unit 140, and the operation control unit 150. Specifically, each unit of the capsule endoscope 10 is connected to the power unit 160 via the power supply control unit 170 such that a power from the power unit 160 can be supplied to each unit or cut off. Under the control by the power supply control unit 170, the power unit 160 supplies the drive power to each unit. Once, under the power supply control unit 170, switching is performed between the state in which the drive power from the power unit 160 is supplied to the state in which the drive power from the power unit 160 is cut off in response to a detection signal from the control signal detecting unit 190, the power supply control unit 170 outputs a feedback signal to the control signal detecting unit 190.

Furthermore, the power supply-control unit 170 includes an oscillation circuit, such as a crystal oscillator that generates a reference clock of a predetermined frequency, and a 1/4 prescaler that generates a sampling clock having a 1/4 frequency of that of the reference clock. While the power is on, the power supply control unit 170 outputs the reference clock and the sampling clock to the control signal detecting unit 190.

The magnetic switch 180 is a main switch of the capsule endoscope 10. Based on an external magnetic signal, an open state and a close state are switched. The magnetic switch 180 is maintained in the close state while being applied with an external magnetic field with a predetermined intensity or more. When the intensity of the external magnetic field decreases, the close state is switched to the open state. The magnetic switch 180 generates a pulse signal based on the magnetic field applied to the magnetic switch 180, and outputs the pulse signal to the control signal detecting unit 190.

The control signal detecting unit 190 includes a power-on signal detector 191 that detects an on pattern signal (power-on signal) as an instruction for supplying a power; and a power-off signal detector 193 that detects an off pattern signal (power-off signal) assigned as an instruction for cutting off the power.

The power-on signal detector 191 serves as a pattern determining unit, and it detects an output pattern of a control signal from the magnetic switch 180 while the power is off. When the output pattern is identical with the on pattern, the power-on signal detector 191 outputs a detection signal to the power supply control unit 170. The on pattern is a signal pattern previously assigned for power-on. For example, a single pulse is assigned as the on pattern. In response to the magnetic signal externaly input by moving a magnet close to or apart from the magnetic switch 180, the state in which the drive power from the power unit 160 is cut off is switched to the state in which the drive power from the power unit 160 is supplied. The sate in which the drive power is supplied is maintained until the power-off signal detector 193 detects the power-off signal.

The power-off signal detector 193 detects an output pattern of control signals from the magnetic switch 180 while the power is on. When the output pattern is identical with the off pattern, the power-off signal detector 193 outputs a detection signal to the power supply control unit 170. The off pattern is a signal pattern previously assigned for power-off. For example, the off pattern consists of a combination of pulse signals of different widths based on the sampling cycle of the sampling clock. Therefore, for example, by inputting an external magnetic signal of the off pattern from the outside of the subject to the magnetic switch 180, the state in which the drive power from the power unit 160 is supplied is switched to the state in which the drive power from the power unit 160 is cut off. The state in which the drive power is cut off is maintained until the power-on signal detector 191 detects the power-on signal.

The control signal detecting unit 190 includes a switch circuit 194 that connects any one of the power-on signal detector 191 and the power-off signal detector 193 to the power supply control unit 170. In the initial state, the switch circuit 194 connects the power-on signal detector 191 and the power supply control unit 170. Upon receiving the feedback signal supplied from the power supply control unit 170, the switch circuit 194 switches the connection. Specifically, the switch circuit 194 connects the power-on signal detector 191 and the power supply control unit 170 to each other while the power is off, and connects the power-off signal detector 193 and the power supply control unit 170 to each other while the power is on. The switch circuit 194 allows the control signal detecting unit 190 to selectively output the detection signal output from the power-on signal detector 191 or the detection signal output from the power-off signal detector 193 to the power supply control unit 170.

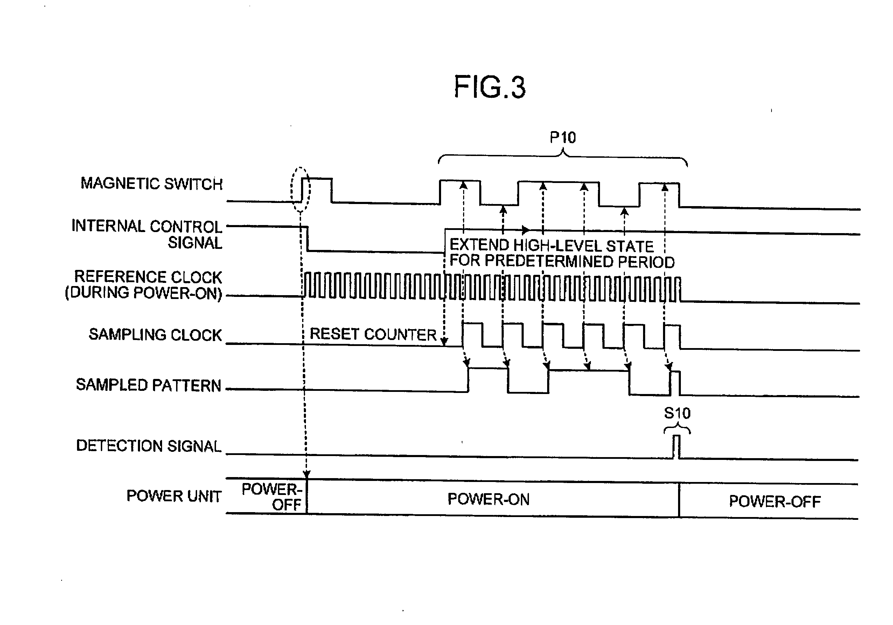

FIG. 3 is a timing chart for explaining an operation of the control signal detecting unit 190 according to the first embodiment. As shown in FIG. 3, once the magnetic switch 180 detects the external magnetic signal and outputs the control signal (single pulse signal) while the power is off, the power-on signal detector 191 detects the control signal as the power-on signal. As a result, the detection signal from the power-on signal detector 191 is input to the power supply control unit 170. Under the control of the power supply control unit 170, the state in which the drive power from the power unit 160 is supplied is achieved, i.e., the drive power is supplied to each unit of the capsule endoscope 10. When the state in which the drive power from the power unit 160 is supplied is achieved, outputting the reference clock is started and a counter of the sampling clock is reset. In addition, an internal control signal for masking the control signal, which is output from the magnetic switch 180 when power is on, for a predetermined period is set to a low level.

On the other hand, once the magnetic switch 180 detects the external magnetic signal and outputs the control signal and the pulse rises, the internal control signal increases to a high level. The high level state of the internal control signal is extended for each predetermined period at the timing of the rising edge of the pulse of the control signal from the magnetic switch 180. When the internal control signal shifts to the high-level state, the counter of the sampling clock is reset and outputting the sampling clock having a frequency of one-fourth of that of the reference clock is started. At the timing of the rising edge of the sampling clock, the control signal from the magnetic switch 180 is sampled. The sampled signal is stored in, for example, a shift register, and is compared with the off pattern. FIG. 3 shows an example where the control signal according to the off pattern is output. Because an output pattern P10 of the control signal from the magnetic switch 180 is identical with the off pattern, the power-off signal detector 193 detects the control signal as the power-off signal. As a result, a detection signal S10 from the power-off signal detector 193 is input to the power supply control unit 170. Under the control of the power supply control unit 170, the state in which the drive power from the power unit 160 is cut off is achieved, i.e., the power supply to each unit of the apparatus is cut off.

According to the first embodiment explained above, different signal patterns can be assigned as an instruction for supplying the power and an instruction for cutting off the power. In response to the single pulse signal output from the magnetic switch 180 while the power is off, the state in which the drive power from the power unit 160 is cut off can be switched to the state in which the drive power from the power unit 160 is supplied. Accordingly, the power can be supplied easily using the simple signal pattern. On the other hand, while the power is on, on the condition that the output pattern of control signals output from the magnetic switch 180 is identical with the off pattern consisting of the pulse signals of different widths, which is assigned for power-off, the state in which the drive power from the power unit 160 is supplied can be switched to the state in which the drive power from the power unit 160 is cut off. This prevents the magnetic switch 180 from erroneously operating while the power is on when the magnetic signal is input to the magnetic switch 180 using the external magnetic field. For example, even when the capsule endoscope 10 is under the environment that an external magnetic field such as a revolving magnetic field is appropriately applied to guide the capsule endoscope 10, the power supply is not cut off as long as the output pattern of control signals from the magnetic switch 180 is not identical with the off pattern. Accordingly, switching between supplying and cutting off the power in the capsule endoscope 10 can be assuredly performed with the simple configuration.

### Second Embodiment

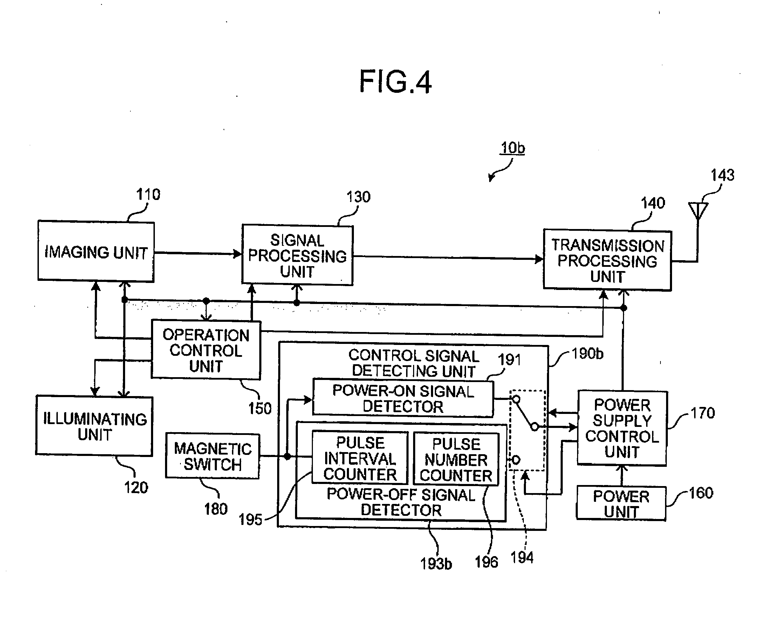

A second embodiment is explained below. FIG. 4 is a block diagram of a functional configuration of a capsule endoscope 10b according to the second embodiment. The same constituents as those of the first embodiment are denoted by the same reference numerals.

As shown in FIG. 4, the capsule endoscope 10b includes the imaging unit 110, the illuminating unit 120, the signal processing unit 130, the transmission processing unit 140, the transmitting antenna 143, the operation control unit 150, the power unit 160, the power supply control unit 170, the magnetic switch 180, and a control signal detecting unit 190b.

In the capsule endoscope 10b, as the first embodiment, the power-on signal detector 191 of the control signal detecting unit 190b detects a single pulse signal, which is input from the magnetic switch 180 while the power is off, as a power-on signal and outputs a detection signal to the power supply control unit 170.

A power-off signal detector 193b serves as a pulse number determining unit, and it detects an output pattern of control signals from the magnetic switch 180 while the power is on. When the output pattern is identical with an off pattern, the power-off signal detector 193b outputs the detection signal to the power supply control unit 170. In the second embodiment, for example, a successive pulse signal consisting of 15 pulse signals is assigned as the off pattern. The number of pulses of the successive pulse signal is not limited to this. The power-off signal detector 193b includes a pulse interval counter 195 and a pulse number counter 196.

The pulse interval counter 195 serves as an interval detecting unit, and it detects a pulse interval between control signals output by the magnetic switch 180 while the power is on. Specifically, the pulse interval counter 195 operates in synchronization with, for example, a reference clock. By counting an interval between the timing of the rising edge of the pulse of the control signal from the magnetic switch 180 and the timing of the rising edge of the pulse of the next control signal, the pulse interval counter 195 detects the output interval between control signals. A maximum value "F" is set as the initial counter value of the pulse interval counter 195. The counter value is reset to "0" at the timing of the rising edge of a pulse of the control signal from the magnetic switch 180. When the pulse interval counter 195 counts up to the maximum value "F", the pulse interval counter 195 holds the counter value as "F" until the timing of the rising edge of the pulse of the next control signal. Based on the output intervals detected by the pulse interval counter 195, it is determined whether the control signals are the successive pulse signal. When the counter value is "1" to "E" at the timing of the rising edge of the pulse of the control signal, it is determined that the successive control signals are continuous pulse signals. When the counter value is "F" at that timing, it is determined that the successive control signals are not continuous pulse signals.

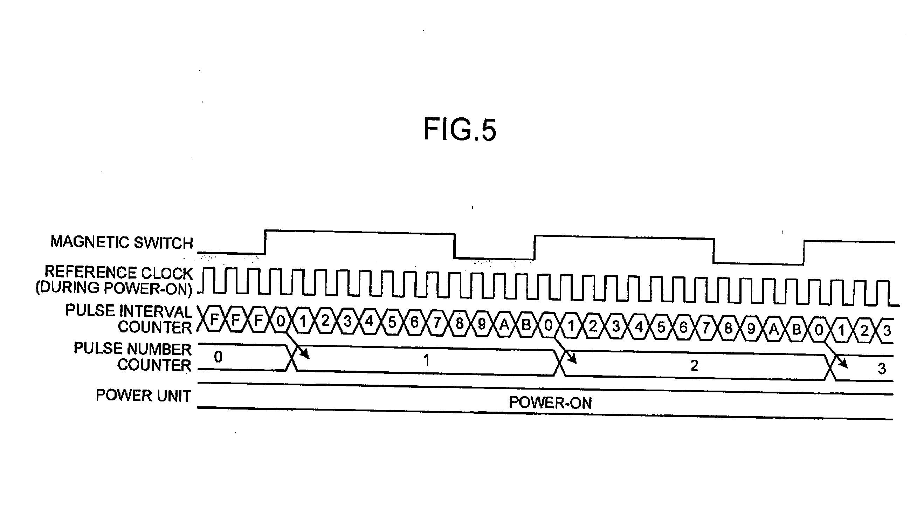

The pulse number counter 196 serves as a pulse number counting unit, and it counts the number of pulses successively output by the magnetic switch 180 while the power is on. The pulse number counter 196 operates depending on the counter value of the pulse interval counter 195 at the timing in synchronization with, for example, the reference clock. The pulse number counter 196 counts up the counter value when the counter value of the pulse interval counter 195 is "0". On the other hand, when the counter value of the pulse interval counter 195 is "F", the pulse number counter 196 resets the counter value of the pulse interval counter 195 to "0" after determining whether the counter value of the pulse number counter 196 is "F". A lower limit of pulse interval can be set to prevent the pulse number counter 196 from counting a pulse with an interval smaller than the predetermined interval among pulses of control signals output by the magnetic switch 180. As shown in FIG. 5, the pulse interval counter 195 counts up at the timing in synchronization with the reference clock. If the pulse number counter 196 is configured to count up when the count value of the pulse interval counter 195 is, for example, "7", the pulse number counter 196 does not count a small pulse whose value counted by the pulse interval counter 195 is "0" to "6".

FIGS. 5 to 7 are timing charts for explaining an operation of the power-off signal detector 193b according to the second embodiment. As shown in FIG. 5, once the magnetic switch 180 detects the external magnetic signal and outputs the control signal and the pulse rises while the power is on, the pulse interval counter 195 sets the counter value from "F" to "0", and counts up the counter value in synchronization with the reference clock. Upon receiving the counter value "0" output from the pulse interval counter 195, the pulse number counter 196 counts up the counter value and outputs "1". Once the next control signal is output from the magnetic switch 180 and a pulse rises, the pulse interval counter 195 sets the counter value to "0", and counts up the counter value again. Upon receiving the counter value "0" output from the pulse interval counter 195, the pulse number counter 196 counts up the counter value and outputs "2".

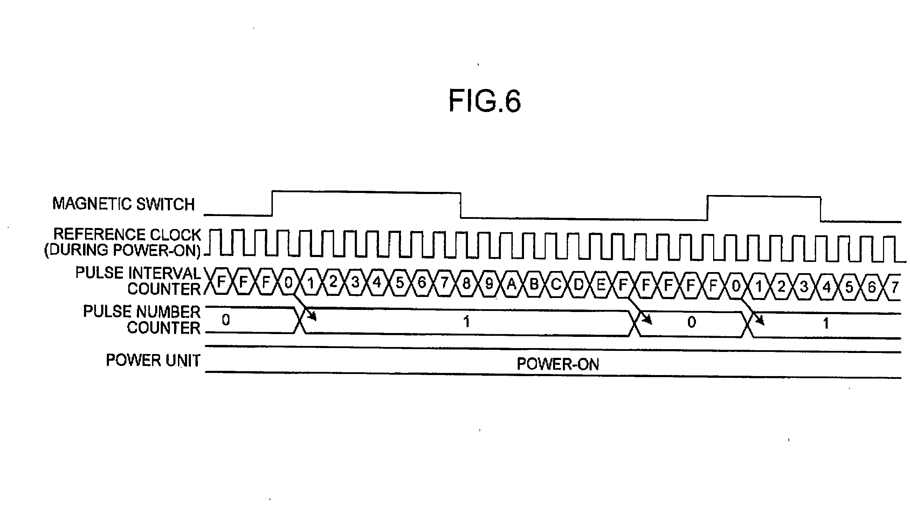

On the other hand, as shown in FIG. 6, when the counter value of the pulse interval counter 195 having started counting up in response to the rising edge of a pulse of a control signal from the magnetic switch 180, the pulse interval counter 195 holds the counter value "F". Upon receiving the counter value "F" output from the pulse interval counter 195, the pulse number counter 196 resets the pulse number counter value to "0".

As explained, when the pulse of the next control signal rises before the counter value of the pulse interval counter 195 reaches "F", it is determined that the successive control signals are continuous pulse signals and the counter value of the pulse number counter 196 is updated. On the other hand, when the pulse of the next control signal rises after the counter value of the pulse interval counter 195 reaches "F", it is determined that the successive control signals are not continuous pulse signals and the counter value of the pulse number counter 196 is reset.

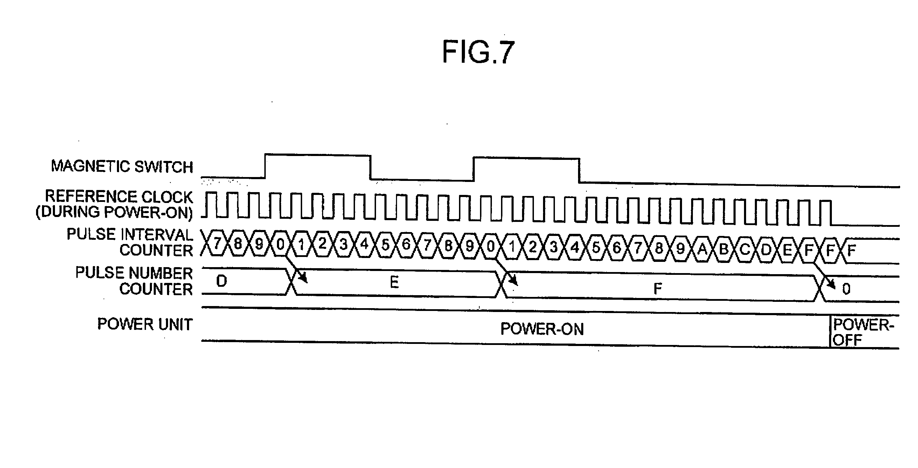

As shown in FIG. 7, when the pulse number counter 196 counts up the counter value to "F" and the counter value of the pulse interval counter 195 reaches "F", the power-off signal detector 193b detects a series of control signals input from the magnetic switch 180 as the power-off signal identical with the off pattern. As a result, the detection signal is input from the power-off signal detector 193b to the power supply control unit 170. Under the control of the power supply control unit 170, the state in which the drive power from the power unit 160 is cut off is achieved, i.e., the power supply to each unit of the apparatus is cut off.

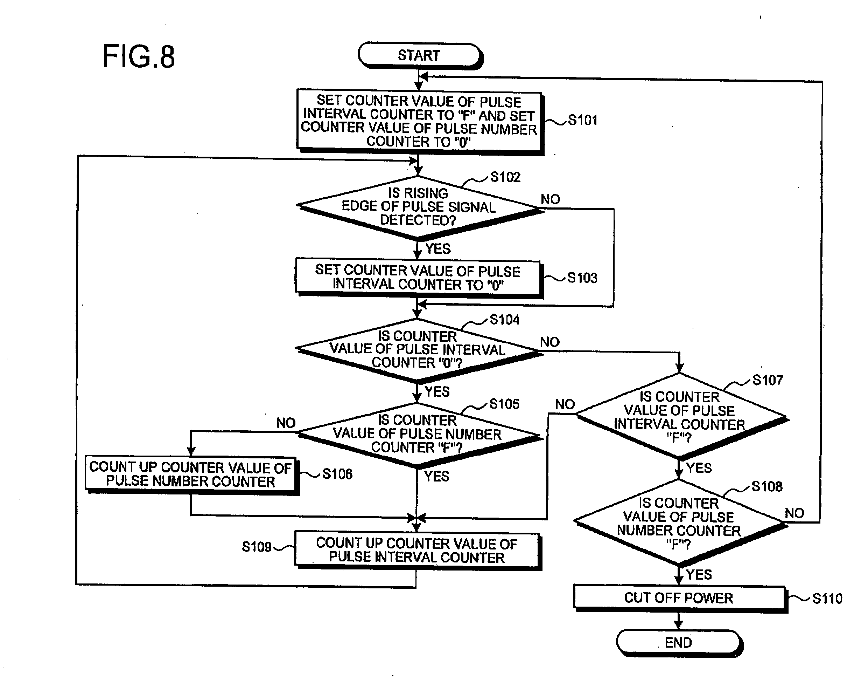

FIG. 8 is a flowchart for explaining a flow of the operation for cutting off the power performed by the capsule endoscope 10b according to the second embodiment. As shown in FIG. 8, the counter value of the pulse interval counter 195 is previously initialized to "F", and the counter value of the pulse number counter 196 is previously initialized to "0" (step S101). When a pulse signal is input from the magnetic switch 180 and the power-off signal detector 193b detects a rising edge of the pulse signal (YES at step S102), the counter value of the pulse interval counter 195 is reset to "0" (step S103). Thereafter, the process goes to step S104 and the counter value of the pulse interval counter 195 is determined. On the other hand, when the power-off signal detector 193b does not detect a rise in the pulse signal (NO at step S102), the counter value of the pulse interval counter 195 is not changed. The process goes to step S104 and the counter value of the pulse interval counter 195 is determined.

When it is determined, after the rising edge of the pulse signal, that the counter value of the pulse interval counter 195 is "0" (YES at step S104), the process goes to step S105 and the counter value of the pulse number counter 196 is determined. When the counter value of the pulse number counter 196 has not reached "F" (NO at step S105), the counter value of the pulse number counter 196 is counted up (step S106). Thereafter, the counter value of the pulse interval counter 195 is counted up (step S109) and the process goes back to determination on whether a rising edge of the pulse signal (step S102) is detected. On the other hand, when the counter value of the pulse number counter 196 has reached "F" (YES at step S105), the counter value of the pulse interval counter 195 is counted up while the counter value of the pulse number counter 196 is held at "F" (step S109).

When the counter value of the pulse number counter 196 is not "0" (NO at step S104), the process goes to step S107 and it is determined whether the value of the pulse interval counter 195 is "F". When the counter value of the pulse interval counter 195 is not "F" (NO at step S107), the counter value of the pulse interval counter 195 is counted up (step S109), and the process goes back to the determination (step S102) on whether a rising edge of the pulse signal is detected. On the other hand, when the counter value of the pulse interval counter 195 is "F" (YES at step S107), the process goes to step S108 and the counter value of the pulse number counter 196 is determined. When the counter value of the pulse number counter 196 is not "F" (NO at step S108), the process goes back to the processing (step S101) for initializing the counter value. When the counter value of the pulse number counter 196 is "F" (YES at step S108), it is determined that the predetermined number of pulses are input and the power is cut off (step S110).

According to the second embodiment explained above, while the power is on, on the condition that the output pattern of the control signals from the magnetic switch 180 is identical with the successive pulse signal serving as the off pattern assigned for port off, the drive power from the power unit 160 can be switched from the state of being supplied to the state of being blocked, which achieves the effect as that of the first embodiment.

As the on pattern for power-on, a signal pattern constituted of a combination of pulse signals of different widths or a predetermined number of successive pulse signals can be assigned. In this case, when the output pattern of a control signal from the magnetic switch 180 identical with the on pattern is detected while the power is off, the state in which the drive power from the power unit 160 is supplied is achieved, i.e., the drive power is supplied to each unit of the capsule endoscope.

According to the first and second embodiments, the case is explained where switching is performed between the drive power from the power unit 160 is supplied and the state in which the drive power from the power unit 160 is cut off based on the output pattern of the control signals corresponding to the magnetic signals input to the magnetic switch 180. This can be similarly applied to the case where the operation mode of the capsule endoscope is switched. In this case, a unique signal pattern is assigned to each operation mode. When an output pattern of control signals identical with the signal pattern is detected, a corresponding operation mode is set. Alternatively, a signal pattern different from the on pattern for power-on and the off pattern for power-off can be assigned to each operation mode. This makes it possible to set the operation mode of the capsule endoscope without erroneously switching the state in which the drive power from the power unit 160 is supplied and the state in which the drive power from the power unit 160 is cut off.

In each of the first and second embodiments, the case is explained where the in-vivo information acquiring apparatus according to the present invention is applied to the capsule endoscope that acquires image information as in-vivo information of the interior of a subject body. Alternatively, for example, it can be similarly applied to another in-vivo information acquiring apparatus that includes a sensor that acquires in-vivo temperature information, in-vivo pressure information, pH information, position information etc.

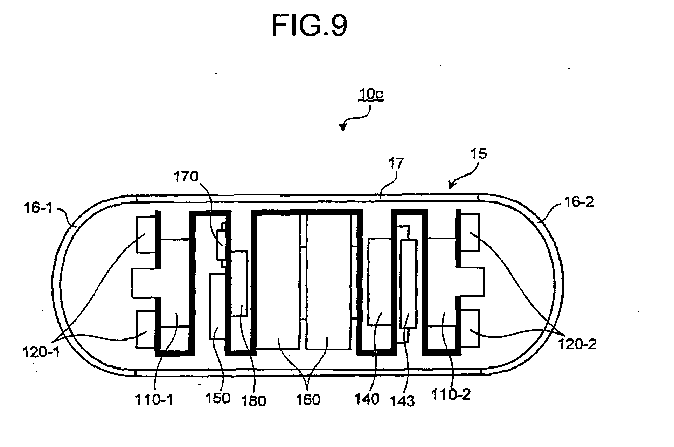

In each of the first and second embodiments, is explained the capsule endoscope that includes a pair of the imaging unit and the illuminating unit. Alternatively, it can be similarly applied to a capsule endoscope that includes two pairs of the imaging unit and the illuminating unit. FIG. 9 is a schematic diagram of a structure of a capsule endoscope 10c that includes two pairs of an imaging unit and an illuminating unit. The constituents same as those of the first embodiment are denoted by the same reference numerals.

The capsule endoscope 10c includes pairs of an imaging unit and an illuminating unit. Each of the pairs is arranged on each tip portion. The capsule endoscope 10c is configured to pick up image information about the front and back sides of the interior of the body of a subject in the moving direction. In other words, as shown in FIG. 9, the capsule endoscope 10c includes a capsule-shaped casing 15 containing imaging units 110-1 and 110-2, illuminating units 120-1 and 120-2, the transmission processing unit 140, the transmitting antenna 143, the operation control unit 150, the power unit 160, the magnetic switch 180, and the power supply control unit 170.

The casing 15 is formed by connecting approximately semi-sphere front covers 16-1 and 16-2 to a cylindrical body cover 17. Each of the front covers 16-1 and 16-2 is formed of a transparent member, and it functions as an optical window. Specifically, the front cover 16-1 allows a light from the illuminating unit 120-1 opposed to the front cover 16-1 in the casing 15 to reach the outside of the casing 15 and guides the reflected light into the casing 15. Similarly, the front cover 16-2 allows a light from the illuminating unit 120-2 opposed to the front cover 16-2 in the casing 15 to reach the outside of the casing 15 and guides the reflected light into the casing 15.

### INDUSTRIAL APPLICABILITY

As described above, an in-vivo information acquiring apparatus and a power supply control method according to the present invention is useful for assuredly switching supplying and cutting off the power in the in-vivo information acquiring apparatus having been introduced into the subject with the simple configuration.

## Claims

1. An in-vivo information acquiring apparatus comprising:
an information acquiring unit that acquires in-vivo information;
a power source that supplies a power to the information acquiring unit;
a magnetic sensor unit that detects a magnetic signal externally input to the magnetic sensor unit, and outputs a control signal corresponding to a state in which the magnetic signal is detected;
a pulse number counting unit that counts the number of pulses of pulse signals from the magnetic sensor unit;
a pulse number determining unit that determines whether the number of pulses counted by the pulse number counting unit is not smaller than a predetermined number; and
a power cut control unit that switches a state in which the power from the power source is supplied to the information acquiring unit to a state in which the power from the power source to the information acquiring unit is cut off.

2. The in-vivo information acquiring apparatus according to claim 1, further comprising an interval detecting unit that detects an interval between outputs of pulse signals from the magnetic sensor, wherein
the pulse number counting unit updates the number of outputs of pulse signals when the interval detected by the interval detecting unit does not exceeds a predetermined reference interval.

3. The in-vivo information acquiring apparatus according to claim 2, wherein the pulse number determining unit determines whether the number of pulses counted by the pulse number counting unit is not smaller than the predetermined number at timing at which the interval detected by the interval detecting unit reaches the predetermined reference interval.

4. The in-vivo information acquiring apparatus according to claim 2, wherein the interval detecting unit includes a counter.

5. The in-vivo information acquiring apparatus according to claim 1, further comprising:
a pattern determining unit that determines a pattern of pulse signals input to the pattern determining unit; and
a power supply control unit that switches the state in which the power from the power source to the information acquiring unit is cut off to the state in which the power from the power source is supplied to the information acquiring unit when a predetermined pattern of pulse signals is input to the pattern determining unit.

6. The in-vivo information acquiring apparatus according to claim 5, wherein the pattern is a single pulse.

7. The in-vivo information acquiring apparatus according to claim 1, wherein the magnetic sensor includes a magnetic switch.

8. A power supply control method performed by an in-vivo information acquiring apparatus including an information acquiring unit that acquires in-vivo information and a power source that supplies a power to the information acquiring unit, the power supply control method comprising:
detecting a magnetic signal externally input;
outputting a control signal corresponding to a state in which the magnetic signal is detected;
counting the number of pulse signals;
determining whether the number of pulse signals is not smaller than a predetermined number; and
switching a state in which the power from the power source is supplied to a state in which the power from the power source is cut off when the number of pulse signals is not smaller than the predetermined number.
